# EUROPEAN PATENT APPLICATION

(11) **EP 4 821 913 A1**
(43) Date of publication of application: **14.10.2026**
(21) Application number: 23960449.9
(22) Date of filing: 05.12.2023
(51) Int. Cl.: C07C 227/02, C07C 227/18, C07C 229/32, C07C 253/30, C07C 255/31, C07C 303/32, C07C 309/29

(54) **METHOD FOR SYNTHESIZING MIROGABALIN BESILATE**

(71) Applicant: Zhejiang Tianyu Pharmaceutical Co., Ltd., Huangyan Taizhou Zhejiang 318020 (CN)
(72) Inventor: WANG, Bo, Huangyan Taizhou, Zhejiang 318020 (CN); LV Huacheng, Huangyan Taizhou, Zhejiang 318020 (CN); ZHU, Guorong, Huangyan Taizhou, Zhejiang 318020 (CN); WANG Chen, Huangyan Taizhou, Zhejiang 318020 (CN); LI Diyuan, Huangyan Taizhou, Zhejiang 318020 (CN); TU, Yongjun, Huangyan Taizhou, Zhejiang 318020 (CN)
(74) Representative: Kador & Partner Part mbB
(86) International application number: PCT/CN2023/136393
(87) International publication number: WO 2025/118134

(57) **Abstract**

A method for synthesizing mirogabalin besilate. The method comprises the following steps: subjecting a chiral ketene and cyanoacetate to a condensation reaction under the action of ammonium acetate to obtain an unsaturated cyano ester, then subjecting the unsaturated cyano ester and nitromethane to a condensation reaction to obtain a nitro ester, subjecting the nitro ester to high-temperature de-esterification to obtain a chiral nitronitrile, then subjecting same to nitroreduction to obtain a chiral aminonitrile, subsequently hydrolyzing the chiral aminonitrile to obtain a mirogabalin free alkali, and finally salifying the mirogabalin free alkali and benzenesulfonic acid to obtain mirogabalin besilate. In the present invention, a chiral cyclobutane quaternary carbon center is stereoscopically and specifically constructed to obtain a chiral nitro ester having a single stereoscopic configuration, thereby preventing chiral isomer waste generated by chiral isomer resolution, such that chiral column separation or chiral resolution is not required, the production cost is significantly reduced, and the residue of heavy-metal titanium in a product and environmental pollution caused by solid waste heavy-metal titanium are also prevented.

## Description

### TECHNICAL FIELD

The present invention relates to the field of medicinal chemistry, and particularly to a method for synthesizing mirogabalin besilate.

### BACKGROUD OF THE INVENTION

In January 2019, mirogabalin besilate developed by Daiichi Sankyo Company Limited of Japan was approved for marketing by the Pharmaceuticals and Medical Devices Agency (PMDA) of Japan for the treatment of peripheral neuropathic pain (PNP), including diabetic peripheral neuropathic pain (DPNP) and postherpetic neuralgia (PHN).

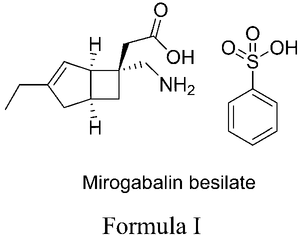

Patent CN101878193 discloses a synthesis route using racemic ketene 2 as a starting material (Reaction Formula II). Firstly, the racemic ketene 2 is subjected to condensation with tert-butyl phosphate to obtain an unsaturated tert-butyl ester A, then the A is subjected to Michael addition with nitromethane to obtain a nitro ester B, and the B is subjected to nitroreduction to obtain a racemic amino acid ester C. Subsequently, the C is separated by a chiral column to obtain a chiral amine D, and the chiral amine is hydrolyzed to obtain a mirogabalin free alkali. Finally, the mirogabalin free alkali is salified with benzenesulfonic acid to obtain mirogabalin besilate. In this route, the stereoselectivity of the quaternary carbon center is poor when the compound B is prepared from the compound A, a stoichiometric amount of chiral isomers are generated, which causes unnecessary contamination, and the isomers must be subjected to chiral separation in subsequent steps. In addition, the hydrolysis of the tert-butyl ester in the compound D needs to be carried out under acidic conditions at high temperatures. Double bond positional isomerization occurs during this process, generating a double bond isomer waste that is difficult to remove, which is unfavorable for obtaining high-purity mirogabalin besilate as an active pharmaceutical ingredient.

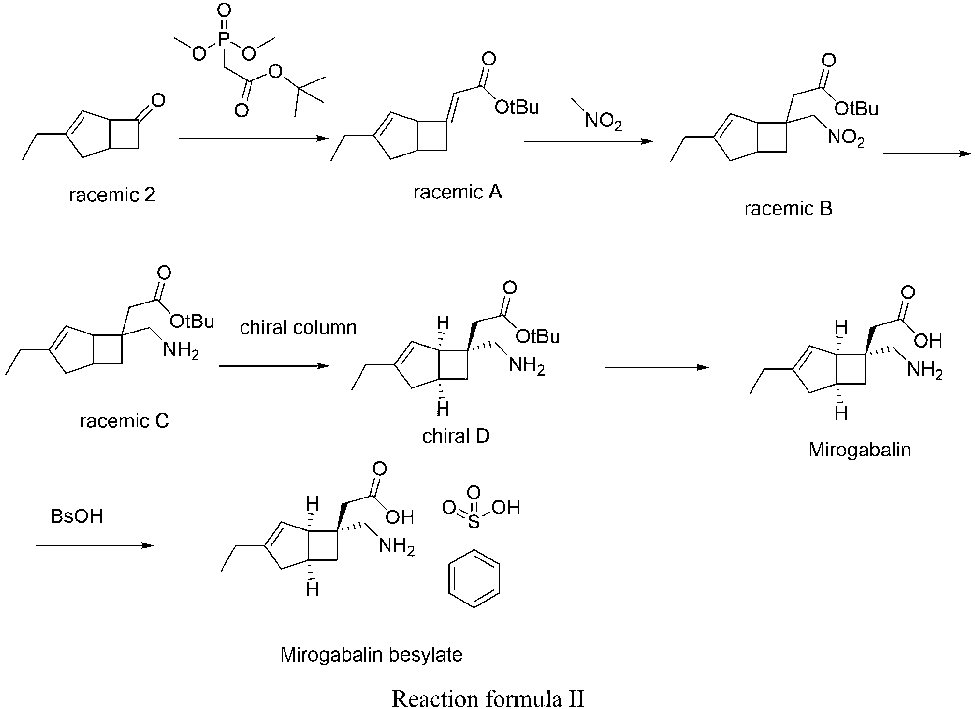

Patent CN104755456 discloses a synthesis route using racemic ketene 2 as a starting material (reaction formula III). Firstly, the racemic ketene 2 is subjected to condensation with diethyl malonate catalyzed by Lewis acid titanium tetrachloride to obtain an unsaturated ester E, then the E is subjected to Michael addition with nitromethane to obtain a nitro ester F, and the F is subjected to high-temperature de-esterification to obtain a nitro ester G. Subsequently, the nitro acid G is hydrolyzed and then resolved with s-phenethylamine to obtain an optically pure nitro acid H, and the nitro acid H is subjected to nitroreduction to obtain a mirogabalin free alkali. Finally, the mirogabalin free alkali is salified with benzenesulfonic acid to obtain mirogabalin besilate. In this route, the two ester groups of the compound E are chemically equivalent when the compound F is prepared from the compound E, a stoichiometric amount of diastereomer wastes are generated, and the isomers must be subjected to chiral resolution in subsequent steps, leading to material waste.

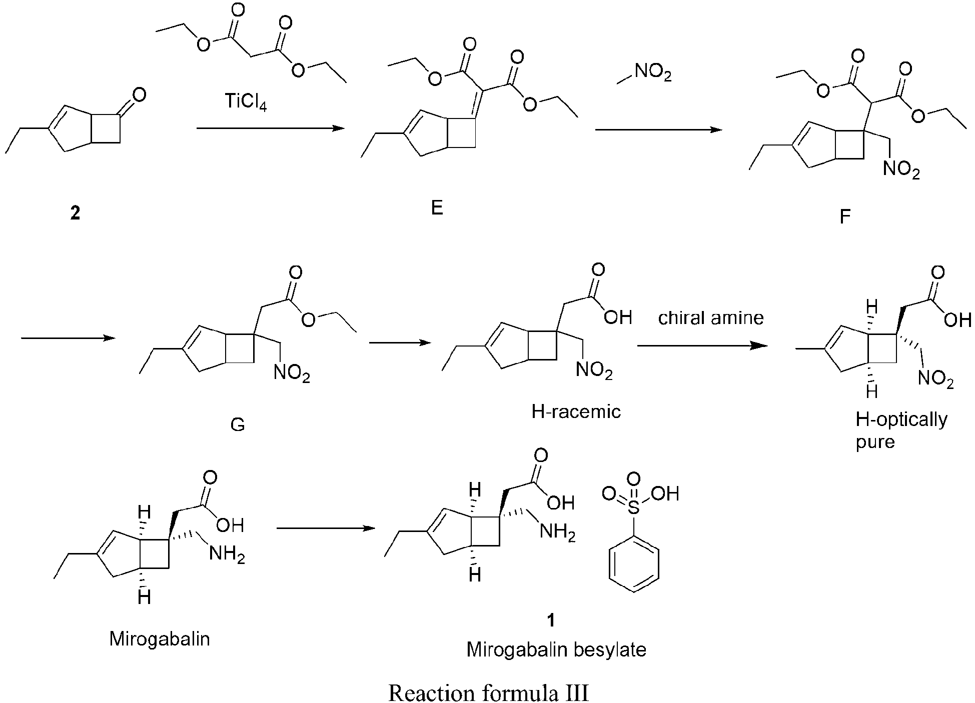

Furthermore, this patent further discloses a method for synthesizing a chiral nitro acid K as a key intermediate of mirogabalin from chiral ketene 2. Firstly, the chiral ketene 2 is subjected to condensation with ethyl phosphate triester (EPE) in the presence of sodium tert-butoxide to obtain an unsaturated ester J, and then the J is subjected to Michael addition with nitromethane to obtain a nitro ester K. In this case, due to poor chiral induction of the ester group, a ratio of diastereomer generated by the quaternary chiral center is 87: 13, and 13% of the chiral isomers generated must be resolved with a chiral amine in subsequent steps to obtain an optically pure nitro acid K.

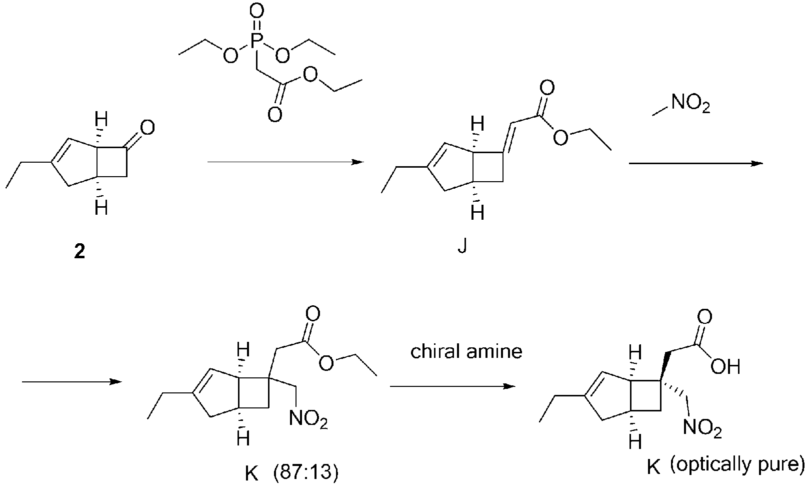

In summary, the prior art has drawbacks, including potential heavy metal residue risks from the use of titanium tetrachloride as a heavy metal reagent, poor chiral control of cyclobutane quaternary carbon center, a requirement for chiral column separation or chiral resolution, and the like, so that it is necessary to explore a concise synthesis route with better chiral control.

### SUMMARY OF THE INVENTION

The present invention provides an improved synthesis method for mirogabalin besilate, which overcomes the deficiencies of the prior art.

The technical core of the present invention is to use a chiral ketene compound 2 as a starting material, and by designing and synthesizing an unsaturated cyanoacetate compound 3 with different substituents on two sides of a double bond, a stereospecific chiral cyclobutane quaternary carbon center is constructed by chiral substrate control when Michael addition is carried out with nitromethane to obtain a compound 4. Details refer to the following technical solution.

In the present invention, the chiral ketene compound 2 is used as the starting material. Firstly, the chiral ketene compound 2 is subjected to condensation with cyanoacetate in the presence of ammonium acetate to obtain an unsaturated cyanoester compound 3, subsequently, the unsaturated cyanoester compound 3 is subjected to condensation with nitromethane to obtain a nitroester compound 4, the nitroester compound 4 is subjected to high-temperature de-esterification to obtain a chiral nitronitrile compound 5, and then the chiral nitronitrile compound 5 was subjected to nitroreduction to obtain a chiral aminonitrile compound 6, subsequently, the chiral aminonitrile compound 6 is hydrolyzed to obtain a mirogabalin free alkali, and finally, the mirogabalin free alkali is salified with benzenesulfonic acid to obtain mirogabalin besilate. 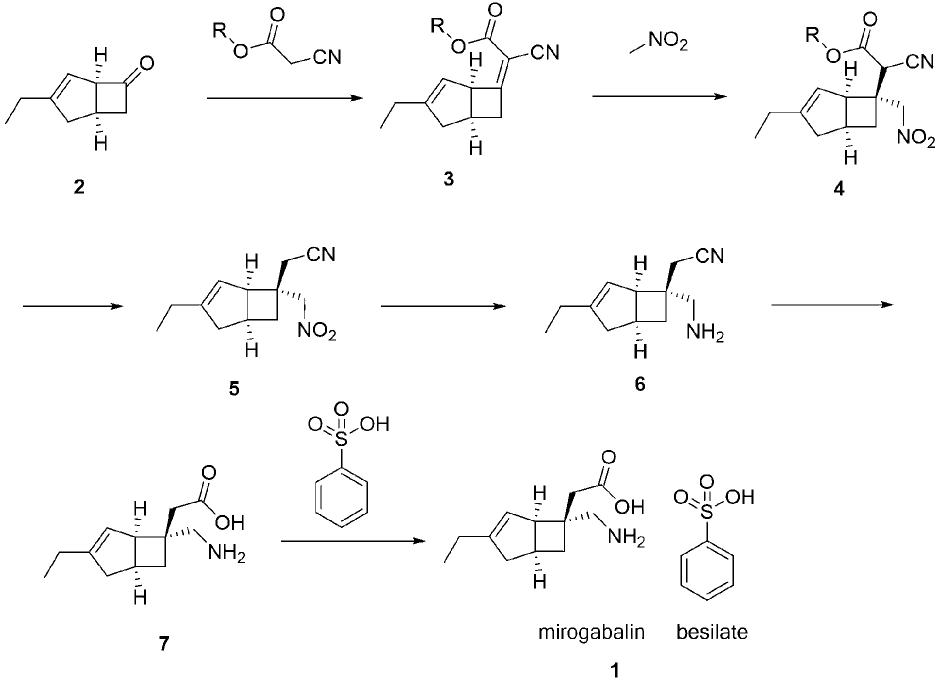 wherein: R is C1-C4 alkyl or benzyl.

Specifically, the synthesis method for mirogabalin besilate of the present invention includes the following steps:
step 1: subjecting a chiral enone compound 2 to condensation with cyanoacetate in the presence of ammonium acetate and acetic acid to obtain an unsaturated cyanoester compound 3;
step 2: under alkaline conditions, subjecting the unsaturated cyanoester compound 3 to condensation with nitromethane to obtain a nitroester compound 4;
step 3: in a high-boiling point solvent, subjecting the nitroester compound 4 to high-temperature de-esterification in the presence of inorganic salt to obtain a chiral nitronitrile compound 5;
step 4: subjecting the chiral nitronitrile compound 5 to nitroreduction in the presence of a reducing agent to obtain a chiral aminonitrile compound 6;
step 5: in an alcoholic solvent, hydrolyzing the chiral aminonitrile compound 6 under alkaline conditions to obtain a mirogabalin free alkali compound 7; and
step 6: salifying the mirogabalin free alkali compound 7 with benzenesulfonic acid to obtain mirogabalin besilate.

According to the synthesis method of the present invention, in the step 1,
the molar ratio of the chiral enone compound 2 to cyanoacetate, acetic acid and ammonium acetate is 1: 1.0-1.5: 0.5-0.8: 0.5-0.8, preferably 1: 1.4: 0.6: 0.6;
the condensation is carried out in toluene, wherein the mass-to-volume ratio (g/mL) of the chiral ketene compound 2 to toluene is 1: 5-10, preferably 1: 6; and
the reaction is carried out at a temperature in a range of 50-110°C, preferably 65-70°C.

According to the synthesis method of the present invention, in the step 2,
the alkali used is selected from triethylamine, tripropylamine, tributylamine or diisopropylethylamine, preferably triethylamine;
the molar ratio of the unsaturated cyanoester compound 3 to nitromethane and alkali is 1: 2.0-4.0: 0.3-3, preferably 1: 3.0: 1.5;
the condensation is carried out in toluene, wherein the mass-to-volume ratio (g/mL) of the unsaturated cyanoester compound 3 to toluene is 1: 5-10, preferably 1: 7; and
the reaction is carried out at a temperature in a range of 0-30°C, preferably 0-5°C.

According to the synthesis method of the present invention, in the step 3,
the inorganic salt is selected from sodium chloride, potassium chloride, sodium sulfate, potassium sulfate, sodium bromide, potassium bromide, or the like, preferably potassium chloride;
the high-boiling point solvent is selected from dimethyl formamide, dimethyl acetamide, dimethyl sulfoxide, toluene, xylene, or the like, preferably dimethyl sulfoxide;
the molar ratio of the nitroester compound 4 to the inorganic salt is 1: 0.4-2.0, preferably 1: 0.7;
the mass-to-volume ratio of the nitroester compound 4 to the high-boiling point solvent is 1: 1-10, preferably 1: 1.4; and
the reaction is carried out at a temperature in a range of 80-140°C, preferably 90-100°C.

According to the synthesis method of the present invention, in the step 4,
the reducing agent may be selected from iron powder/acetic acid, zinc powder/acetic acid, Raney nickel/H₂, Pd/C/H₂, and the like;
when the reducing agent is the iron powder/acetic acid or the zinc powder/acetic acid:
   the molar ratio of the chiral nitronitrile compound 5 to the iron powder or zinc powder and acetic acid is 1: 5-10: 5-15, preferably 1: 6-8: 8-12, and more preferably 1: 6: 10;
   the organic solvent is selected from methanol, ethanol, isopropanol, tert-butanol, and the like, preferably methanol;
   the mass-to-volume ratio (g/mL) of the chiral nitronitrile compound 5 to the organic solvent is 1: 5-12, preferably 1: 6-10, and more preferably 1: 6.8; and
   when the reducing agent is Raney nickel/H₂ or Pd/C/H₂:
      the mass ratio of the chiral nitronitrile compound 5 to the reducing agent is 1: 0.03-0.1, preferably 1: 0.05;
      the organic solvent is selected from methanol, ethanol, isopropanol, tert-butanol, and the like, preferably methanol;
      the mass-to-volume ratio (g/mL) of the chiral nitronitrile compound 5 to the organic solvent is 1: 5-12, preferably 1: 6-10, and more preferably 1: 6.8.

According to the synthesis method of the present invention, in the step 5,
the alkali may be selected from sodium hydroxide, potassium hydroxide or lithium hydroxide;
the molar ratio of the chiral aminonitrile compound 6 to the alkali is 1: 2-15, preferably 1: 5-10, and more preferably 1: 7.5;
the alcoholic solvent is selected from methanol, ethanol, n-propanol, butanol or isopropanol, preferably ethanol;
the mass-to-volume ratio (g/mL) of the chiral aminonitrile compound 6 to the alcoholic solvent is 1: 2-15, preferably 1: 2-8, and more preferably 1: 3; and
the reaction is carried out at a temperature in a range of 60-100°C, preferably 60-70°C.

According to the synthesis method of the present invention, in the step 6,
the mirogabalin free alkali compound 7 is salified with benzenesulfonic acid in water; and
the mass ratio (g/g) of the mirogabalin free alkali compound 7 to water is 1: 6.

In another aspect, when a racemic enone compound 2 is used as a starting material, a racemic aminonitrile compound 6 may be obtained according to the process conditions above, and the aminonitrile compound 6 is resolved with mandelic acid to obtain the optically pure chiral aminonitrile compound 6 (a synthesis route is shown as follows). 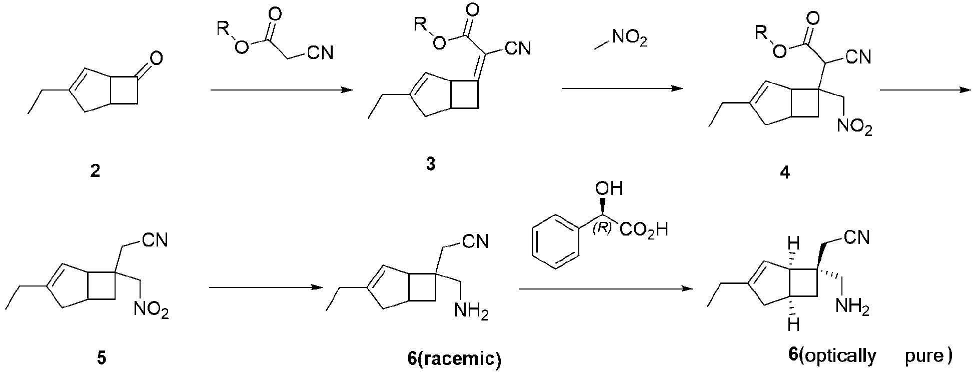 wherein: R is C1-C4 alkyl or benzyl.

Specifically, a synthesis method for the optically pure chiral aminonitrile compound 6 of the present invention includes the following steps:
step 1: subjecting a racemic enone compound 2 to condensation with cyanoacetate in the presence of ammonium acetate and acetic acid to obtain a racemic unsaturated cyanoester compound 3;
step 2: under alkaline conditions, subjecting the racemic unsaturated cyanoester compound 3 to condensation with nitromethane to obtain a racemic nitroester compound 4;
step 3: in a high-boiling point solvent, subjecting the racemic nitroester compound 4 to high-temperature de-esterification in the presence of inorganic salt to obtain a racemic nitronitrile compound 5;
step 4: subjecting the racemic nitronitrile compound 5 to nitroreduction in the presence of a reducing agent to obtain a racemic aminonitrile compound 6; and
step 5: resolving the racemic aminonitrile compound 6 with mandelic acid to obtain the optically pure chiral aminonitrile compound 6.

According to the synthesis method for the optically pure chiral aminonitrile compound 6 of the present invention, the process conditions of step 1 to step 4 are the same as those of step 1 to step 4 in the aforementioned synthesis method for mirogabalin besilate of the present invention.

According to the synthesis method for the optically pure chiral aminonitrile compound 6 of the present invention, in the step 5,
the mass ratio of the racemic aminonitrile compound 6 to mandelic acid is 1: 0.8-0.9; and
mandelic acid used for resolution is D-mandelic acid.

### Beneficial effects

Compared with the prior art, the advantages of the present invention are as follows.
(1) In the prior art, malonate with a symmetric molecular structure is used in the condensation with the enone compound 2, and the chiral control of the quaternary carbon center is poor during the subsequent condensation with the nitromethane; and in the present invention, by designing and synthesizing the unsaturated cyanoacetate compound 3 with different substituents on two sides of the double bond, the chiral induction of the chiral enone substrate is utilized to subject the nitromethane to the Michael addition, so as to stereospecifically construct the chiral cyclobutane quaternary carbon center, so that the chiral nitroester compound 4 with a single stereoscopic configuration is obtained.
(2) In the present invention, by adopting ethyl cyanoacetate as a condensation reagent, a prochiral source is introduced, which realizes the stereospecific construction of the chiral cyclobutane quaternary carbon, and avoids a chiral isomer waste generated by chiral isomer resolution, so that it is not necessary to carry out chiral column separation or chiral resolution, and production costs are greatly reduced.
(3) In the present invention, the ammonium acetate is used in instead of titanium tetrachloride adopted in the prior art as a condensation catalyst, which has a remarkable cost advantage, and avoids a heavy metal titanium residue in the product and environmental pollution caused by a solid heavy metal titanium waste at the same time.

### DESCRIPTION OF EXEMPLARY EMBODIMENTS

The present invention will be described in detail hereinafter with reference to specific embodiments and examples, and the advantages and various effects of the present invention will be presented more clearly. It should be understood by those skilled in the art that these specific embodiments and examples are merely used for illustrating the present invention and are not intended to limit the present invention.

Analytical conditions for the compounds of the present invention are as follows: ¹H NMR and ¹³C NMR are measured with a Bruker Avance 400 nuclear magnetic resonance spectrometer; and HRMS is measured by a Waters Xevo G2-XS QTof high-resolution mass spectrometer, with an ESI source as an ion source.

### Example 1 Preparation of compound 3

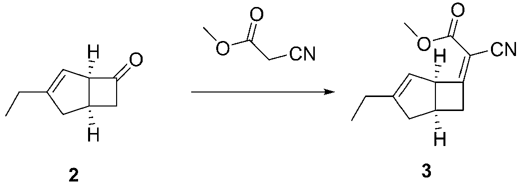

50 g of chiral enone compound 2, 50.9 g of methyl cyanoacetate, 17 g of ammonium acetate, 13.2 g of acetic acid and 300 mL of toluene were added to a reaction vessel. The mixture was heated to 65-70°C and subjected to reflux water separation at the temperature. The reaction was monitored by liquid chromatography until completion (15-20 hours), and the reaction solution was cooled to room temperature and washed twice with 600 mL of water. Organic layers were collected, dried with anhydrous sodium sulfate and filtered, and the filtrate was concentrated to dryness under reduced pressure to obtain 75.7 g of compound 3, with a yield of 95%. HRMS m/z (ESI): C₁₃H₁₆NO₂ [M+H⁺] calcd for: 218.1176, found: 218.1172.

¹H-NMR (400MHz, CDCl₃) δ: 5.32 (m, 1H), 4.16 (br, 1H), 3.79 (s, 3H), 3.49 (m, 1H), 2.99 (m, 1H), 2.84-2.70 (m, 2H), 2.29 (m, 1H), 2.08 (q, J = 6.4Hz, 2H), 1.03 (t, J = 6.4Hz, 3H).

¹³C-NMR (100MHz, CDCl₃) δ: 188.07, 161.65, 149.95, 118.89, 113.83, 99.44, 52.27, 42.41, 40.74, 38.91, 32.70, 24.19, 12.07.

By varying the enone compound 2 and cyanoacetate, the compound 3 was prepared according to the process parameters and the operating steps of Example 1, as shown in the examples in the following table.

| Serial number | Enone compound 2 | Cyanoacetate | Compound 3 (HRMS) | Yield of compound 3 |
|---|---|---|---|---|
| Example 2 | 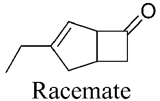 | 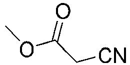 | 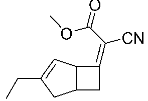 Racemate HRMS m/z (ESI): C₁₃H₁₆NO₂ [M+H⁺] | 94% |
| | | | Calcd for: 218.1176, found: 218.1173. | |
| Example 3 | 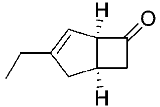 | 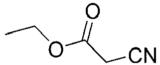 | 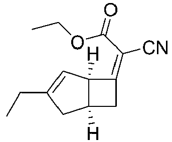 HRMS m/z (ESI): C₁₄H₁₈NO₂ [M+H⁺] | 91% |
| | | | Calcd for: 232.1332, found: 232.1336. | |
| Example 4 | 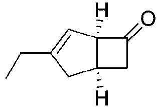 | 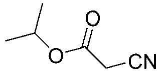 | 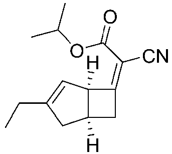 HRMS m/z (ESI): C₁₅H₂₀NO₂ [M+H⁺] | 88% |
| | | | Calcd for: 246.1489, found: 246.1486. | |
| Example 5 | 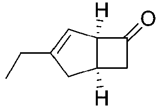 | 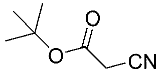 | 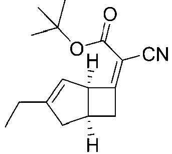 HRMS m/z (ESI): C₁₆H₂₂NO₂ [M+H⁺] | 81% |
| | | | Calcd for: 260.1645, found: 260.1649. | |
| Example 6 | 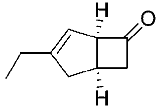 | 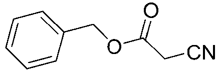 | 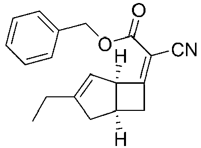 HRMS m/z (ESI): C₁₉H₂₀NO₂ [M+H⁺] | 85% |
| | | | Calcd for: 294.1489, found: 294.1484. | |

Nuclear magnetic resonance data of the compound 3 prepared in Example 3:
¹H-NMR (400MHz, CDCl₃) δ: 5.37 (m, 1H), 4.45 (br, 1H), 4.25 (m, 2H), 3.45 (m, 1H), 3.00 (m, 1H), 2.88-2.74 (m, 2H), 2.30 (m, 1H), 2.12 (q, J = 6.4Hz, 2H), 1.34 (t, J = 6.4Hz, 3H), 1.03 (t, J = 6.4Hz, 3H).
¹³C-NMR (100MHz, CDCl₃) δ: 187.36, 161.15, 149.83, 118.87, 113.80, 99.85, 61.34, 59.29, 42.36, 38.85, 32.63, 24.15, 14.08, 12.03.

### Example 7: Preparation of compound 4

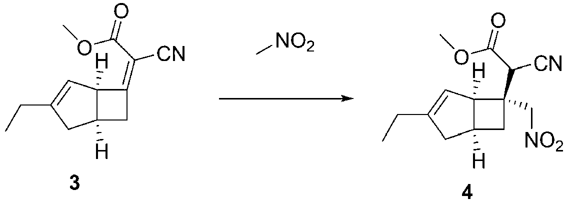

217 g of compound 3, 183 g of nitromethane and 1500 mL of toluene were added to a reaction vessel, and 150 g of triethylamine was slowly added dropwise at 0-5°C. After the dropwise addition was complete, the mixture was allowed to react under thermal insulation for 18-24 hours. After the reaction was complete, the reaction mixture was cooled to 0-5°C, 10% hydrochloric acid was added along with 500 mL of water, and the mixture was stirred for layer separation. The organic layer was washed once with 1000 mL of water, separated and concentrated to dryness under reduced pressure to obtain 250 g of chiral nitroester compound 4, with a yield of 90%. HRMS m/z (ESI): C₁₄H₁₉N₂O₄ [M+H⁺] calcd for:279.1339, found: 279.1332.

¹H-NMR (400MHz, CDCl₃) δ: 5.35 (m, 1H), 4.82-4.72 (m, 2H), 3.97 (m, 1H), 3.75 (s, 1H), 3.21 (m, 1H), 2.89 (m, 2H), 2.54-2.40 (m, 3H), 2.14 (m, 3H), 1.72 (m, 1H), 1.09 (t, J = 6.4Hz, 3H).

¹³C-NMR (100MHz, CDCl₃) δ: 164.51, 153.54, 118.51, 114.16, 79.32, 53.32, 52.59, 45.80, 42.24, 41.48, 34.81, 30.84, 24.40, 12.11.

Example 8: Racemic compound 4 was prepared, with a yield of 88%, by replacing chiral compound 3 with racemic compound 3 according to the operating steps of Example 3.

### Example 9: Preparation of compound 5

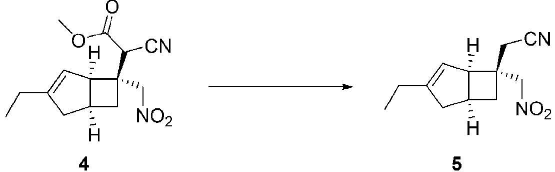

73 g of compound 4, 100 mL of dimethyl sulfoxide (DMSO), 19.8 g of water and 26 g of sodium sulfate were added to a reaction flask. The mixture was heated to 95-100°C and allowed to react under thermal insulation for 18-22 hours. After the reaction was complete, the reaction mixture was cooled to room temperature and filtered, 250 mL of ethyl acetate was added to the filtrate, the filter cake was dried, and the residue was subjected to column chromatography by using ethyl acetate: n-hexane = 1: 10 as a developing solvent to obtain 55 g of compound 5, which was an oily liquid, with a yield of 95% and a purity of 95%. HRMS m/z (ESI): C₁₂H₁₇N₂O₂ [M+H⁺] calcd for:221.1258, found: 221.1252.
[α]²⁵D (C=1, ethanol) = - 124.8 °
¹H-NMR (400MHz, CDCl₃) δ: 5.35 (br, 1H), 4.73 (s, 2H), 3.19 (m, 1H), 2.91 (m, 1H), 2.64 (s, 2H), 2.54 (m, 1H), 2.29 (m, 1H), 2.16 (m, 3H), 1.61 (m, 1H), 1.10 (t, J = 6.4Hz, 3H).
¹³C-NMR (100MHz, CDCl₃) δ: 152.98, 119.27, 117.10, 80.45, 51.67, 43.15, 42.27, 35.39, 30.33, 24.36, 22.59, 12.31.

Example 10: Racemic compound 5 was prepared, with a yield of 88%, by replacing chiral compound 4 with racemic compound 4 according to the operating steps of Example 5.

### Example 11: Preparation of compound 6

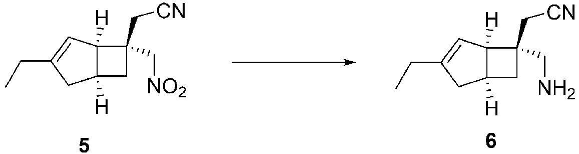

336 g of iron powder, 220 g of compound 5, 600 g of acetic acid and 1500 mL of methanol were added to a reaction vessel. The mixture was then heated to 70-75°C and allowed to react under thermal insulation for 18-22 hours. After the reaction was complete, the reaction mixture was cooled to room temperature and filtered, and the filtrate was concentrated to dryness under reduced pressure to obtain 171 g of compound 6, which was an oily liquid, with a yield of 90%. HRMS m/z (ESI): C₁₂H₁₉N₂ [M+H⁺] calcd for:191.1543, found: 191.1548.
[α]²⁵D (C=1, ethanol ) = - 131 °
¹H-NMR (400MHz, CDCl₃) δ: 5.35 (br, 1H), 4.79 (br, 2H), 3.69 (m, 2H), 2.96(m, 1H), 2.69 (m, 2H), 2.42 (m, 2H), 2.08-1.94 (m, 5H), 1.61 (m, 1H), 1.04 (t, J = 6.4Hz, 3H).
¹³C-NMR (100MHz, CDCl₃) δ: 165.58, 147.65, 123.01, 69.01, 54.54, 48.34, 42.13, 41.72, 39.01, 30.92, 23.82, 12.33.

### Example 12: Preparation of compound 6

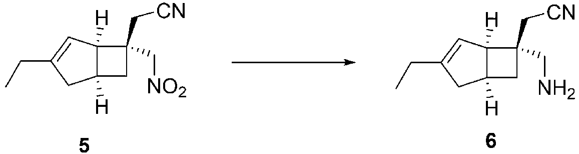

11 g of palladium on carbon (5%), 220 g of compound 5 and 1500 mL of methanol were added to a pressure vessel, and hydrogen was then added until a pressure reached 0.3 MPa. The mixture was allowed to react at room temperature under thermal insulation for 4 hours. After the reaction was complete, the reaction mixture was filtered, and the filtrate was concentrated to dryness under reduced pressure to obtain 180 g of compound 6, which was an oily liquid, with a yield of 95%.
[α]²⁵D (C=1, ethanol ) = - 130 °
HRMS m/z (ESI): C₁₂H₁₉N₂ [M+H⁺] calcd for:191.1543, found: 191.1546.

Example 13: Racemic compound 6 was prepared, with a yield of 92%, by replacing chiral compound 5 with compound 5 according to the operating steps of Example 11.

### Example 14: Chiral resolution of racemic compound 6

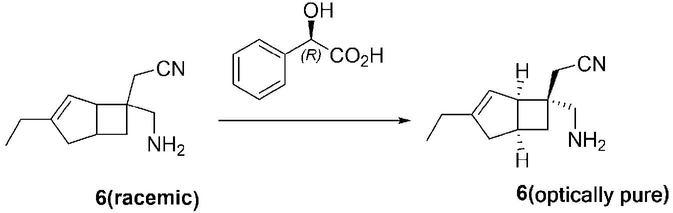

190 g of racemic compound 6, 155 g of D-mandelic acid and 1000 mL of acetonitrile were added to a reaction vessel, and the mixture was heated to 50°C and stirred until complete dissolution. Subsequently, the mixture was slowly cooled to 0°C within 3 hours, a large amount of white crystal was precipitated out, and the resulting crystal was filtered and dried under vacuum to obtain D-mandelate of an optically pure compound 6. Subsequently, the D-mandelate of the optically pure compound 6 was dissolved in ethanol, and pH was adjusted to 9 with an aqueous sodium hydroxide solution. Ethanol was removed from the residue, and the mixture was extracted with 500 mL of ethyl acetate. The organic layer was concentrated under reduced pressure to obtain 76 g of optically pure compound 6, with a yield of 40%.
[α]²⁵D (C=1, ethanol ) = - 130°

### Example 15: Preparation of mirogabalin free alkali

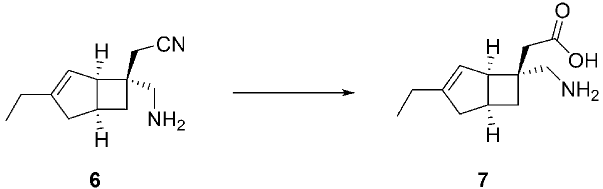

At -5-0°C, 190 g of compound 6, 570 mL of ethanol and 1500 g of 20% aqueous sodium hydroxide solution were added to a reaction vessel, and the mixture was then heated to 60-70°C for reaction. The reaction was monitored by liquid chromatography until completion (24 hours), and pH of the synthetic solution was adjusted to 6-7 with 10% hydrochloric acid. A large amount of white solid was precipitated out and filtered, and the resulting solid was filtered and dried under vacuum to obtain 199 g of mirogabalin, with a yield of 95%. HRMS m/z (ESI): C₁₂H₂₀NO₂ [M+H⁺] calcd for: 210.1489, found: 210.1485.

¹H-NMR (400MHz, CD₃OD) δ: 5.38 (dd, J = 1.7, 3.7 Hz, 1H), 3.18 (d, J = 13.0 Hz, 1H), 3.14 (d, J = 13.0 Hz, 1H), 3.10 (m, 1H), 2.85 (q, J = 7.5 Hz, 1H), 2.51 (d, J = 16.2 Hz, 1H), 2.46-2.53 (m, 1H), 2.46 (d, J = 16.2 Hz, 1H), 2.14 (q, J = 7.4 Hz, 2H), 2.08-2.03 (m, 2H), 1.48 (dd, J = 7.5, 12.5 Hz, 1H), 1.10 (t, J = 7.4 Hz, 3H).

### Example 16: Preparation of mirogabalin besilate

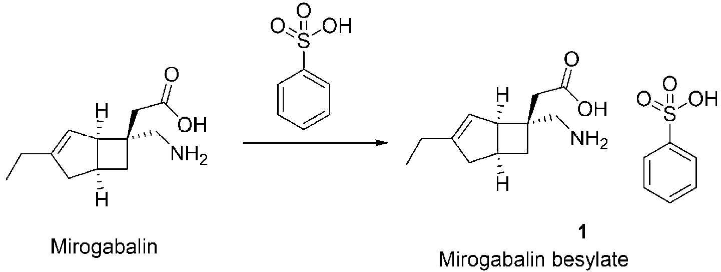

200 g of mirogabalin, 162 g of benzenesulfonic acid and 1200 mL of purified water were added to a reaction vessel, and the mixture was then heated to 70-75°C and stirred until full dissolution. Subsequently, the mixture was slowly cooled to 0°C within 3 hours, a large amount of white crystal was precipitated out, and the resulting crystal was filtered and dried under vacuum to obtain 309 g of mirogabalin besilate (white crystal), with a yield of 88%.

The foregoing provides detailed description of the specific embodiments of the present invention, which are merely preferred exemplary embodiments of the present invention. Technically speaking, any optimizations of the reaction conditions in the implementation steps based on the synthesis route of the present invention and any methodological improvements made to obtain the intermediates involved in the present invention shall also be deemed to fall within the scope of protection of the present invention. Therefore, the present invention is not limited to the specific embodiments described above. For those skilled in the art, any equivalent modifications and substitutions made to the present invention shall also fall within the scope of the present invention.

## Claims

1. A synthesis method for mirogabalin besilate, with a reaction formula shown as follows: 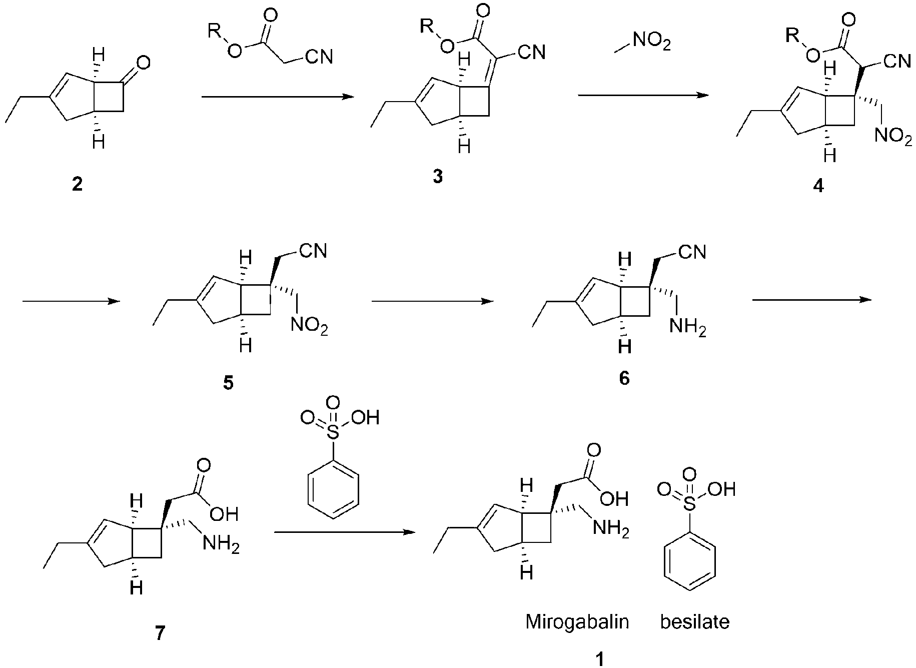 wherein: R is C1-C4 alkyl or benzyl. wherein, the synthesis method specifically comprises the following steps:
step 1: subjecting a chiral enone compound 2 to condensation with cyanoacetate in the presence of ammonium acetate and acetic acid to obtain an unsaturated cyanoester compound 3;
step 2: under alkaline conditions, subjecting the unsaturated cyanoester compound 3 to condensation with nitromethane to obtain a nitroester compound 4;
step 3: in a high-boiling point solvent, subjecting the nitroester compound 4 to high-temperature de-esterification in the presence of inorganic salt to obtain a chiral nitronitrile compound 5;
step 4: subjecting the chiral nitronitrile compound 5 to nitroreduction in the presence of a reducing agent to obtain a chiral aminonitrile compound 6;
step 5: in an alcoholic solvent, hydrolyzing the chiral aminonitrile compound 6 under alkaline conditions to obtain a mirogabalin free alkali compound 7; and
step 6: salifying the mirogabalin free alkali compound 7 with benzenesulfonic acid to obtain mirogabalin besilate.

2. The synthesis method for mirogabalin besilate according to claim 1, wherein, in the step 1, the molar ratio of the chiral enone compound 2 to cyanoacetate, acetic acid and ammonium acetate is 1: 1.0-1.5: 0.5-0.8: 0.5-0.8.

3. The synthesis method for mirogabalin besilate according to claim 2, wherein, in the step 1, the molar ratio of the chiral enone compound 2 to cyanoacetate, acetic acid and ammonium acetate is 1: 1.4: 0.6: 0.6.

4. The synthesis method for mirogabalin besilate according to claim 1, wherein, in the step 1, the reaction is carried out at a temperature in a range of 50-110°C.

5. The synthesis method for mirogabalin besilate according to claim 4, wherein, in the step 1, the reaction is carried out at a temperature in a range of 65-70°C.

6. The synthesis method for mirogabalin besilate according to claim 1, wherein, in the step 2, the alkali used is selected from triethylamine, tripropylamine, tributylamine or diisopropylethylamine.

7. The synthesis method for mirogabalin besilate according to claim 1, wherein, in the step 2, the molar ratio of the unsaturated cyanoester compound 3 to nitromethane and alkali is 1: 2.0-4.0: 1.0-1.5.

8. The synthesis method for mirogabalin besilate according to claim 1, wherein, in the step 2, the reaction is carried out at a temperature in a range of 0-30°C.

9. The synthesis method for mirogabalin besilate according to claim 8, wherein, in the step 2, the reaction is carried out at a temperature in a range of 15-20°C.

10. The synthesis method for mirogabalin besilate according to claim 1, wherein, in the step 3, the high-boiling point solvent is selected from dimethyl formamide, dimethyl acetamide, dimethyl sulfoxide, toluene or xylene.

11. The synthesis method for mirogabalin besilate according to claim 1, wherein, in the step 3, the reaction is carried out at a temperature in a range of 80-140°C.

12. The synthesis method for mirogabalin besilate according to claim 1, wherein, in the step 3, the inorganic salt is selected from sodium chloride, potassium chloride, sodium sulfate, potassium sulfate, sodium bromide or potassium bromide.

13. The synthesis method for mirogabalin besilate according to claim 1, wherein, in the step 4, the reducing agent is selected from iron powder/acetic acid, zinc powder/acetic acid, Raney nickel/hydrogen, or palladium on carbon/hydrogen.

14. The synthesis method for mirogabalin besilate according to claim 1, wherein, in the step 5, the alkali used is selected from sodium hydroxide, potassium hydroxide or lithium hydroxide.

15. The synthesis method for mirogabalin besilate according to claim 1, wherein, in the step 5, the reaction is carried out at a temperature in a range of 60-100°C.

16. The synthesis method for mirogabalin besilate according to claim 1, wherein, in the step 5, the alcoholic solvent is selected from methanol, ethanol, n-propanol, butanol or isopropanol.

17. The synthesis method for mirogabalin besilate according to claim 1, wherein, in the step 5, the mass-to-volume ratio of the chiral aminonitrile compound 6 to the alcoholic solvent is 1: 2-15.

18. A synthesis method for an optically pure chiral aminonitrile compound 6, comprising the following steps: using a racemic enone compound 2 as a starting material, preparing a racemic aminonitrile compound 6 according to the process conditions of step 1 to step 4 in claim 1, and subsequently resolving the racemic aminonitrile compound 6 with mandelic acid to obtain the optically pure chiral aminonitrile compound 6, wherein the synthesis route is shown as follows: 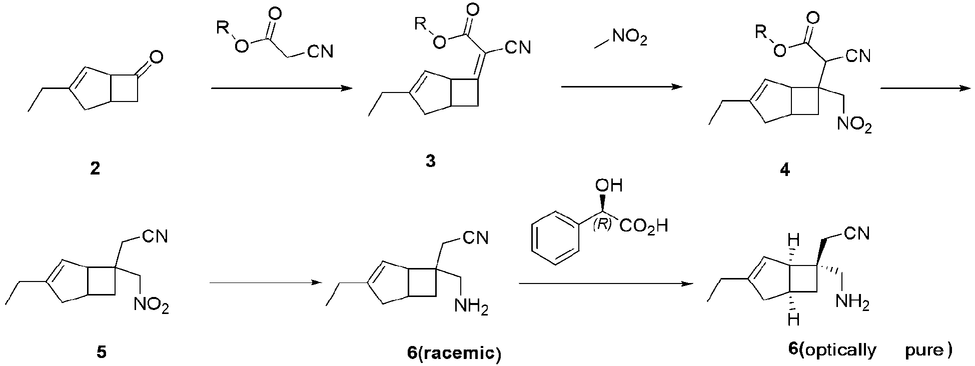 wherein: R is C1-C4 alkyl or benzyl.

19. A key ester intermediate for synthesis of mirogabalin, with a structure shown as follows: 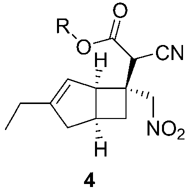 wherein: R is C1-C4 alkyl or benzyl.

20. A key cyano intermediate for synthesis of mirogabalin, with a structure shown as follows:
